# EUROPÄISCHE PATENTSCHRIFT

(11) **EP 2 408 765 B1**
(45) Veröffentlichungstag und Bekanntmachung des Hinweises auf die Patenterteilung: **23.01.2013**
(21) Anmeldenummer: 10715090.6
(22) Anmeldetag: 12.03.2010
(51) Int. Cl.: C07D 405/12

(54) **NEUES VERFAHREN ZUR HERSTELLUNG VON ENAMINOCARBONYL-VERBINDUNGEN**
NOVEL METHOD FOR PRODUCING ENAMINOCARBONYL COMPOUNDS
NOUVEAU PROCÉDÉ DE PRÉPARATION DE COMPOSÉS ÉNAMINOCARBONYLÉS

(30) Priorität: 16.03.2009 EP 09155202
(43) Veröffentlichungstag der Anmeldung: 25.01.2012
(73) Patentinhaber: Bayer CropScience AG, 40789 Monheim (DE)
(72) Erfinder: LUI, Norbert, 51519 Odenthal (DE); HEINRICH, Jens-Dietmar, 51399 Burscheid (DE)
(86) Internationale Anmeldenummer: PCT/EP2010/001577
(87) Internationale Veröffentlichungsnummer: WO 2010/105779

(56) Entgegenhaltungen:
- EP-A- 0 539 588
- WO-A-93/22305
- WO-A-99/38846
- US-A- 5 905 090
- ROBERT A. MACK, PATRICK J. CARROLL, VASSIL ST. GERORGIEV: "On the mechanism of a novel 3(2H).-furanone-2(5H)-furanone rearrangement" J. HETEROCYCL. CHEM., Bd. 25, Nr. 2, 1988, Seiten 603-606, XP009123390
- VASSIL ST. GEORGIEV, ROBERT A. MACK, DAVID J. WALTER, LESLEY A. RADOV, JANE E. BAER: "Drug-induced modifications of the immune response 4-(Arylamino)-2,5-dihydro-2-oxo-N-(trans-2 -phenylcyclopropyl)furan-3-carboxamides as novel antiallergic compounds" HELVETICA CHIMICA ACTA, Bd. 70, 1987, Seiten 1526-1530, XP002548067
- GIORGIO BERTOLINI, MARIO AQUINO, MAURO BIFFI, GAETANO D'ATRI ET AL.: "A new rational hypothesis for the pharmacophore of the active metabolite of leflunomide, a potent immunosuppressive drug" J. MED. CHEM., Bd. 40, 1997, Seiten 2011-2016, XP002548068

## Beschreibung

Die vorliegende Erfindung betrifft ein Verfahren zur Herstellung von 4-Amino-but-2-enoliden.

Bestimmte substituierte Enaminocarbonyl-Verbindungen sind als insektizid wirksame Verbindungen aus der EP 0 539 588 A1 bekannt. Darüber hinaus beschreiben auch die internationalen Patentanmeldungen WO 2007/115644, WO 2007/115643 und WO 2007/115646 entsprechende insektizid wirksame Enaminocarbonyl-Verbindungen.

Im Allgemeinen werden Enaminocarbonylverbindungen aus Tetronsäure und einem Amin gemäß dem nachfolgenden Schema 1 synthetisiert. Diese Vorgehensweise ist beispielsweise in EP 0 539 588 A1 sowie in Heterocycles Vol. 27, Nr. 8, Seite 1907 bis 1923 (1988) beschrieben.

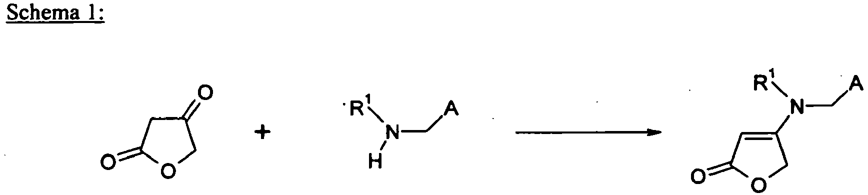

Nachteilig an diesem Verfahren ist insbesondere, dass man wasserfreie Tetronsäure als Ausgangsverbindung benötigt, deren Herstellung aufwendig und kostenintensiv ist.

So wird Tetronsäure im Allgemeinen ausgehend von Acetessigester über eine Bromierung und anschließende Hydrierung hergestellt (vgl. Synthetic Communication, 11(5), Seiten 385 bis 390 (1981)). Die Gesamtausbeute an Tetronsäure ausgehend von Acetessigester ist dabei kleiner als 40 %, was das Verfahren unter industriellem Gesichtspunkt wenig attraktiv macht.

In der CH-PS 503 722 ist ein weiteres Verfahren zur Herstellung von Tetronsäure beschrieben. Dabei wird 4-Chloracetessigester mit einem aromatischen Amin zum 3-Arylaminocrotonlacton umgesetzt und anschließend wird die Tetronsäure durch Behandlung mit Mineralsäuren freigesetzt. Der Nachteil an diesem Verfahren besteht darin, dass die Isolierung der Tetronsäure nur durch Hochvakuum-Sublimation möglich ist, was auch dieses Verfahren unter industriellem Gesichtspunkt wenig attraktiv macht.

Ein weiteres Verfahren zur Herstellung von Tetronsäure ist in der EP 0 153 615 A beschrieben, in welchem von 2,4-Dichloracetessigestern ausgegangen wird. Dieses ebenfalls mehrstufige und aufwändige Verfahren liefert die gewünschte Verbindung ebenfalls nur mit einer mäßigen Gesamtausbeute von 65 %.

In Tetrahedron Letters, Nr. 31, Seiten 2683 und 2684 (1974) ist die Herstellung von Tetronsäure und einer entsprechenden Enaminocarbonylverbindung beschrieben. Die dort beschriebene Synthese ist in folgendem Schema 2 wiedergegeben. Als Edukt wird dabei Acetylendicarbonsäuredimethylester eingesetzt.

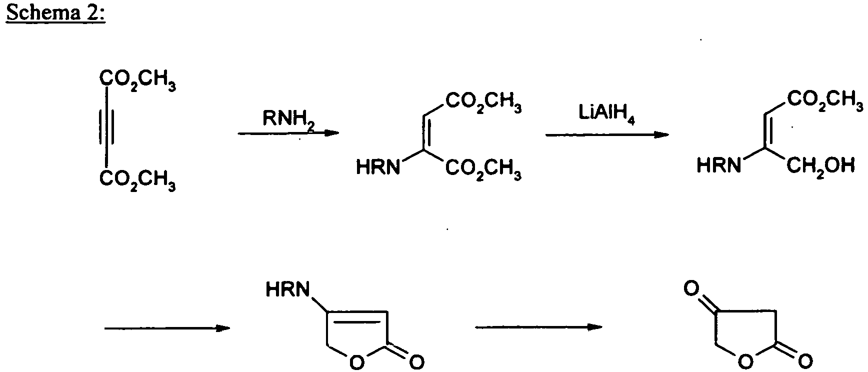

Nachteilig an diesem Verfahren ist die geringe Gesamtausbeute von nur 30 % sowie das Erfordernis, kostenintensive Edukte, beispielsweise Lithiumaluminiumhydrid (LiA1H₄), als Reagenzien verwenden zu müssen.

Ferner ist aus dem Stand der Technik ein Verfahren zur Herstellung von Enaminocarbonyl-Verbindungen ausgehend von Methyltetronat bekannt (J. Heterocyclic Chem., 21, 1753 (1984)). Für dieses Verfahren wird als Ausgangsmaterial der kostenintensive 4-Brom-3-methoxy-but-3-encarbonsäureester verwendet.

Ein weiteres Verfahren geht von einem 4-Chloracetessigester aus, der mit Aminen umgesetzt wird (Heterocycles, Vol. 27, Nr. 8, 1988, Seiten 1907 bis 1923). Die Reaktion zum Aminofuran wird in einem Schritt durchgeführt. Dabei wird das Amin mit Eisessig zu einer Lösung von 4-Chloracetessigester in Benzol gegeben und die resultierende Mischung wird mehrere Stunden unter Rückfluss erhitzt. Die Ausbeuten an 4-Methylamino-2(5H)-furanon in dieser Synthese betragen nur 40 %.

Aus EP 0 123 095 A ist ein Verfahren bekannt, in welchem Tetronsäureamid aus 3-Amino-4-acetoxycrotonsäureester hergestellt wird. 3-Amino-4-acetoxycrotonsäureester ist kostenintensiv und aufwändig herzustellen, so dass eine wirtschaftliche Synthese mit diesem Verfahren nicht möglich ist.

Ein weiteres Verfahren zur Herstellung von Tetronsäure ausgehend von Malonestern und Chloracetylchlorid ist aus J. Chem. Soc., Perkin Trans. 1 (1972), Nr. 9/10, Seiten 1225 bis 1231 bekannt. Dieses Verfahren liefert die gewünschte Zielverbindung mit einer Ausbeute von nur 43 %.

In der vorstehend erwähnten, Internationalen Patentanmeldung WO 2007/115644 wird die Herstellung von Enaminocarbonyl-Verbindungen, beispielsweise von 4-[[(6-Chlorpyridin-3-yl)methyl](3,3-dichlorprop-2-en-1-yl)amino]furan-2(5H)-on durch Umsetzung von 4-[[(6-Chlorpyridin-3-yl)methyl]amino]furan-2(5H)-on mit 3-Brom-1,1-dichlorprop-1-en, beschrieben (vgl. Herstellungsbeispiel, Verfahren 2, Beispiel (3)). Die WO 2007/115644 beschreibt auch die Herstellung von Enaminocarbonyl-Verbindungen, beispielsweise von 4-[[(6-Chlorpyridin-3-yl)methyl](2-Fluorethyl)amino]furan-2(5H)-on durch Umsetzung von 4-[[(2-Fluorethyl)amino]furan-2(5H)-on mit 2-Chlor-5-chlormethyl-pyridin (vgl. Herstellungsbeispiele, Verfahren 3, Beispiel (4)). Die Reaktionen werden vorzugsweise mit Hydriden des Lithiums oder Natriums durchgeführt. Diese Substrate sind im Allgemein kostenintensiv und gleichzeitig aus Sicherheitsgründen nur schwer zu handhaben.

In WO 2009/036899, die die Priorität der Europäischen Patentanmeldung Nr. 07116639 beansprucht, werden Enaminocarbonylverbindungen beispielsweise ausgehend von 4-(Methoxycarbonyl)-5-oxo-2,5-dihydrofuran-3-ol und einem Amin hergestellt. 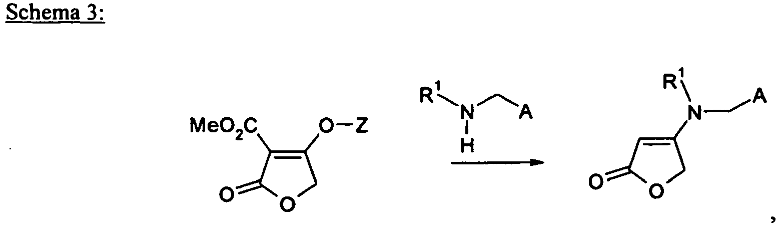 wobei
- R¹: für Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyloxyalkyl, Halogencyclo-alkylalkyl oder Arylalkyl steht;
- Z: für Wasserstoff, Alkalimetall oder Erdalkalimetall steht; und
- A: Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder für

Pyrimidinyl, Pyrazolyl, Thiophenyl; Oxazolyl, Isoxazolyl, 1,2,4-Oxadiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder für 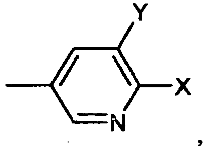 in welchem
- X: für Halogen, Alkyl oder Halogenalkyl steht und
- Y: für Halogen, Alkyl, Halogenalkyl, Halogenalkoxy, Azido oder Cyan steht.

Ausgehend von diesem Stand der Technik ergibt sich als Aufgabe der vorliegenden Erfindung, ein alternatives Verfahren zur Herstellung von Enaminocarbonyl-Verbindungen bereitzustellen, welches vorzugsweise einfach und kostengünstig durchzuführen ist. Die mit diesem angestrebten Verfahren erhältlichen Enaminocarbonyl-Verbindungen sollen dabei vorzugsweise mit hoher Ausbeute und hoher Reinheit erhalten werden. Insbesondere soll das angestrebte Verfahren den Erhalt der gewünschten Zielverbindungen ohne die Notwendigkeit komplexer Aufreinigungsmethoden ermöglichen.

Gelöst wird diese Aufgabe durch ein neues Verfahren zur Herstellung von Enaminocarbonyl-Verbindungen der allgemeinen Formel (I):

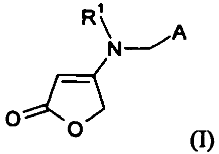

Das erfindungsgemäße Verfahren ist dadurch gekennzeichnet, dass man Verbindungen der allgemeinen Formel (II) 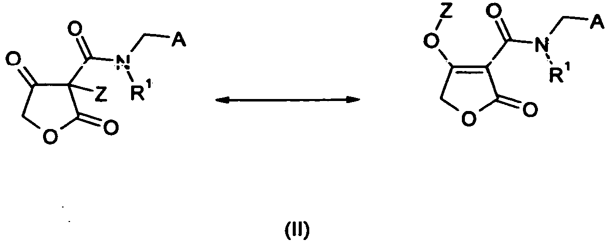 zu Verbindungen der Formel (I) umsetzt, wobei
- R¹: für Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyloxyalkyl, Halogencycloalkylalkyl, Aryl oder Arylalkyl steht;
- Z: für Wasserstoff, Alkalimetall oder Erdalkalimetall steht; und
- A: für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder für

Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder für 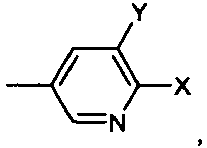 in welchem
- X: für Halogen, Alkyl oder Halogenalkyl steht und
- Y: für Halogen, Alkyl, Halogenalkyl, Halogenalkoxy, Azido oder Cyan steht.

Erfindungsgemäß ist somit vorgesehen, dass die gewünschten Enaminocarbonyl-Verbindungen der allgemeinen Formel (I) durch eine Umsetzung der entsprechenden Verbindungen der allgemeinen Formel (II) hergestellt werden. Die gewünschten Enaminocarbonyl-Verbindungen der allgemeinen Formel (I) werden unter den erfindungsgemäßen und weiter unten näher spezifizierten bevorzugten Reaktionsbedingungen mit guten Ausbeuten in hoher Reinheit erhalten, womit das erfindungsgemäße Verfahren die oben genannten Nachteile der Verfahren des Standes der Technik überwindet. Die gewünschten Verbindungen werden dabei in einer Reinheit erhalten, welche eine umfangreiche Aufarbeitung des unmittelbaren Reaktionsprodukts im Allgemeinen nicht erforderlich macht.

Bevorzugte, besonders bevorzugte und ganz besonders bevorzugte Bedeutungen des in den oben erwähnten allgemeinen Formeln (I) und (II) aufgeführten Restes A und R¹ werden im Folgenden erläutert.
- A: wird bevorzugt ausgewählt aus der Gruppe bestehend aus 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl und 5-Difluormethyl-6-iod-pyrid-3-yl.
- R¹: wird bevorzugt ausgewählt aus Wasserstoff, Alkyl, Halogenalkyl, Alkenyl, Halogenalkenyl, Alkinyl, Cycloalkyl, Cycloalkylalkyl, Halogencycloalkyl, Halogencycloalkylalkyl und Alkoxyalkyl.
- Z: wird bevorzugt ausgewählt aus der Gruppe bestehend aus Alkalimetallen und Wasserstoff;
- A: wird besonders bevorzugt ausgewählt aus der Gruppe, bestehend aus 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlor-pyrid-3-yl.
- R¹: wird besonders bevorzugt ausgewählt aus der Gruppe bestehend aus Methyl, Ethyl, Propyl, Vinyl, Allyl, Propargyl, Cyclopropyl, Alkoxyalkyl, 2-Fluor-ethyl, 2,2-Difluor-ethyl und 2-Fluor-cyclopropyl.
- Z: wird besonders bevorzugt ausgewählt aus der Gruppe bestehend aus Wasserstoff, Natrium und Kalium;
- A: wird ganz besonders bevorzugt ausgewählt aus der Gruppe bestehend aus 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl.
- R¹: wird ganz besonders bevorzugt ausgewählt aus der Gruppe bestehend aus Methyl, Ethyl, n-Propyl, n-Prop-2-enyl, n-Prop-2-inyl, Cyclopropyl, Methoxyethyl, 2-Fluorethyl und 2,2-Difluor-ethyl.
- Z: wird ganz besonders bevorzugt ausgewählt aus der Gruppe bestehend aus Natrium und Wasserstoff;

In einer bevorzugten Ausführungsförm der vorliegenden Erfindung werden in dem erfindungsgemäßen Verfahren Ausgangsverbindungen der allgemeinen Formel (II) eingesetzt, in welchen die Substituenten A, Z und R¹ die vorstehend erwähnten bevorzugten Bedeutungen aufweisen, wobei die bevorzugten, besonders bevorzugten und ganz bevorzugten Bedeutungen der Substituenten kombiniert werden können.

In einer besonders bevorzugten Ausführungsform der vorliegenden Erfindung werden in dem erfindungsgemäßen Verfahren Ausgangsverbindungen der allgemeinen Formel (II) eingesetzt, in welchen die Substituenten A, Z und R¹ die vorstehend erwähnten besonders bevorzugten Bedeutungen aufweisen, wobei die bevorzugten, besonders bevorzugten und ganz bevorzugten Bedeutungen der Substituenten kombiniert werden können.

In einer ganz besonders bevorzugten Ausführungsform der vorliegenden Erfindung werden in dem erfindungsgemäßen Verfahren Ausgangsverbindungen der allgemeinen Formel (II) eingesetzt, in welchen die Substituenten A, Z und R¹ die vorstehend erwähnten ganz besonders bevorzugten Bedeutungen aufweisen, wobei die bevorzugten, besonders bevorzugten und ganz bevorzugten Bedeutungen der Substituenten kombiniert werden können.

Im Rahmen der vorliegenden Erfindung wird - unabhängig von den einzelnen oben erwähnten bevorzugten, besonders bevorzugten und ganz besonders bevorzugten Bedeutungen - den einzelnen verwendeten Resten im Allgemeinen folgende Bedeutung zugewiesen:

Sofern nichts anderes erwähnt wird unter dem Begriff "Alkyl", entweder in Alleinstellung oder aber in Kombination mit weiteren Begriffen, wie beispielsweise Halogenalkyl, Alkoxyalkyl, Cycloalkylalkyl, Halogencycloalkylalkyl und Arylalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste, speziell Methyl und Ethyl.

Sofern nichts anderes erwähnt wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Sofern nichts anderes erwähnt wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Sofern nichts anderes erwähnt wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Sofern nichts anderes erwähnt wird unter dem Begriff "Aryl" erfindungsgemäß ein aromatischer Rest mit 6 bis 14 Kohlenstoffatomen, vorzugsweise Phenyl, verstanden.

Sofern nichts anderes erwähnt wird unter dem Begriff "Arylalkyl" eine Kombination von erfindungsgemäß definierten Resten "Aryl" und "Alkyl" verstanden, wobei der Rest im Allgemeinen über die Alkylgruppe gebunden wird. Beispiele hierfür sind Benzyl, Phenylethyl oder α-Methylbenzyl, wobei Benzyl besonders bevorzugt ist.

Sofern nichts anderes erwähnt werden unter dem Begriff "durch Halogen substituierte Reste" beispielsweise Halogenalkyl, einfach oder mehrfach bis zur maximal möglichen Substituentenzahl halogenierte Reste verstanden. Bei mehrfacher Halogenierung können die Halogenatome gleich oder verschieden sein. Halogen steht dabei für Fluor, Chlor, Brom oder Iod, insbesondere für Fluor, Chlor oder Brom.

Sofern nichts anderes erwähnt wird unter dem Begriff "Alkoxy", entweder in Alleinstellung oder aber in Kombination mit weiteren Begriffen, wie beispielsweise Halogenalkoxy, vorliegend ein Rest O-Alkyl verstanden, wobei der Begriff "Alkyl" die oben stehende Bedeutung aufweist.

Gegebenenfalls substituierte Reste können einfach oder mehrfach substituiert sein, wobei bei einer Mehrfachsubstitution die Substituenten gleich oder verschieden sein können.

Ein weiterer Gegenstand der vorliegenden Erfindung sind Verbindungen der Formel (II) 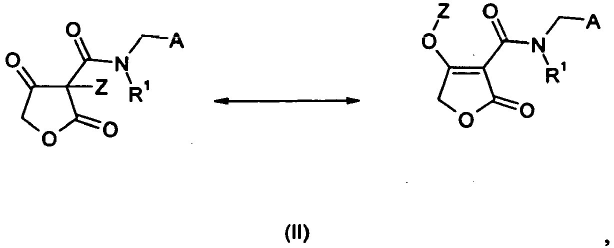 in welcher die Reste A, Z und R¹ wie oben definiert sind.

Die Synthese entsprechender abgewandelter Derivate der Verbindungen der allgemeinen Formel (II) kann gemäß nachfolgendem Schema 4 beispielsweise ausgehend von 2,4-Dioxotetrahydrofuran-3-carboxylaten der allgemeinen Formel (IV) mit Aminen der Formel (III) durchgeführt werden oder nach Literatur hergestellt werden (Bertolini et al., J. Med. Chem. 1997, 40,2011 - 2016, Benary, Bier., 1908, 41, 1943.): 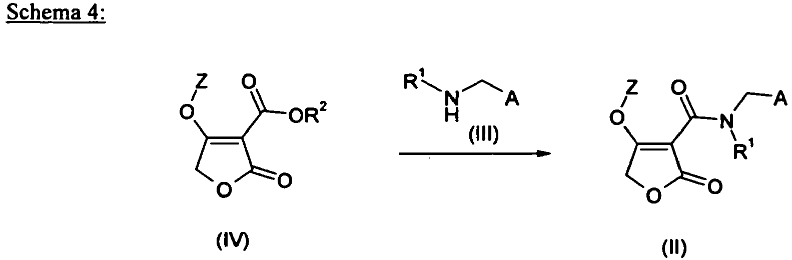 wobei die Reste A, Z und R¹ wie oben definiert sind und R² für Alkyl, Aryl oder Arylalkyl steht.

Die Verbindung (II) kann auch in einer Isomerenform vorliegen.

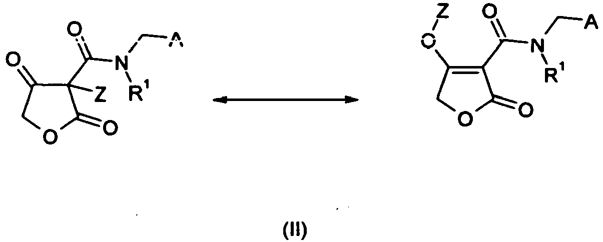

Die als Edukte verwendeten 2,4-Dioxotetrahydrofuran-3-carboxylate der allgemeinen Formel (IV) können nach aus dem Stand der Technik bekannten Verfahren hergestellt werden (R. Anschütz, Ber., 1912, 45, 2374; E. Benary, Ber., 1912, 45, 3682). Die Amine der allgemeinen Formel (III) sind kommerziell erhältlich oder können nach literaturbekannten Verfahren hergestellt werden (vgl. z.B. S. Patai " The Chemistry of Amino Group", Interscience Publishers, New York, 1968).

Die oben dargestellte erfindungsgemäße Umsetzung der Verbindungen der allgemeinen Formel (II) zu Verbindungen der allgemeinen Formel (I) wird in Gegenwart von Lösungsmitteln (Verdünnungsmitteln) durchgeführt. Lösungsmittel werden vorteilhaft in einer solchen Menge eingesetzt, dass das Reaktionsgemisch während des ganzen Verfahrens gut rührbar bleibt. Als Lösungsmittel zur Durchführung des erfindungsgemäßen Verfahrens kommen alle unter den Reaktionsbedingungen inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Ether, wie Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di-n-butylether, Diisobutylether, Diisoamylether, Ethylenglycoldimethylether, Isopropylethylether, Methyl-tert-butylether, Tetrahydrofuran, Methyl-Tetrahydrofuran, Dioxan, Dichlordiethylether; Methyl-THF und Polyether des Ethylenoxids und/oder Propylenoxids; Nitrokohlenwasserstoffe wie Nitromethan, Nitroethan, Nitropropan, Nitrobenzol, Chlomitrobenzol, o-Nitrotoluol; Nitrile wie Acetonitril, Propionitril, Butyronitril, Isobutyronitril, Benzonitril, Phenylnitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, n-Hexan, n-Heptan, n-Oktan, Nonan beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, N,N-Dimethyl-acetamid, *N*-Methyl-formamid, *N*,*N*-Dimethyl-formamid, *N*,*N*-Dipropyl-formamid, *N,N*-Dibutyl-formamid *N-*Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N-*Formyl-piperidin, *N*,*N*'-1,4-Diformyl-piperazin; und aliphatische Alkohole, wie Methanol, Ethanol, n-Propanol und iso-Propanol und n-Butanol.

Die erfindungsgemäße Umsetzung wird vorzugsweise in einem Lösungsmittel durchgeführt, welches ausgewählt ist aus der Gruppe, bestehend aus Dioxan, Butyronitril, Propionitril, Acetonitril, DME, Toluol, Methyl-THF, Dichlorbenzol, Chlorbenzol, n-Heptan, iso-Butanol, n-Butanol, Ethanol, Methyl-tert.-butylether, Isopropylethylether und Mischungen davon.

Die Reaktion kann auch in Gegenwart von Wasser durchgeführt werden.

Die Umsetzung der Verbindungen der allgemeinen Formel (II) wird vorzugsweise in Gegenwart einer Bronstedt-Säure durchgeführt.

Das molare Verhältnis der eingesetzten Brenstedt-Säure zu den Aminen der Formel (III) kann varüeren Vorzugsweise liegt das Verhältnis von Br⌀nstedt-Säure zum Amin der Formel (III) im Bereich von etwa 10 : 0,6 bis etwa 1: 1,5, insbesondere von etwa 5: 0,9 bis 1: 1,2, speziell von etwa 2:1 bis etwa 1: 1,1.

Dabei ist es möglich, sowohl organische als auch anorganische Br⌀nstedt-Säurenzu verwenden. Vorzugsweise werden anorganische Säuren, beispielsweise Phosphorsäure (H₃PO₄), Schwefelsäure (H₂SO₄), Salzsäure (HCl), Bromwasserstoffsäure (HBr), Flusssäure (HF) oder Kaliumhydrogensulfat (KHSO₄), verwendet. Die einzelnen Säuren können dabei sowohl in wasserfreier Form als auch in wasserhaltiger Form, beispielsweise als 85%ige Phosphorsäure oder 37%ige Salzsäure, d.h. insbesondere in Formen, in welchen die Säuren kommerziell erhältlich sind, verwendet werden. Beispiele für geeignete organische Säuren sind Trifluoressigsäure, Essigsäure, Methansulfonsäure und p-Toluolsulfonsäure. Von den zuvor genannten Säuren sind insbesondere Phosphorsäure, Schwefelsäure, Kaliumhydrogensulfat und Trifluoressigsäure bevorzugt.

Die Umsetzung zur Herstellung der Verbindungen der allgemeinen Formel (I) kann im Allgemeinen im Vakuum, bei Normaldruck oder unter Überdruck durchgeführt werden. Die angewendeten Temperaturen können ebenfalls, in Abhängigkeit der verwendeten Substrate, variieren und sind für den Fachmann durch Routineversuche leicht zu ermitteln. Beispielsweise kann die Umsetzung zur Herstellung der Verbindungen der allgemeinen Formel (I) bei einer Temperatur von 20 bis 200 °C, vorzugsweise 20 bis 150°C, durchgeführt werden.

Zum Ende der Reaktion kann das Reaktionswasser durch Destillation eines Teils des Lösungsmittel als Azeotrop entfernt werden. Bei hochsiedenden Lösungsmittel kann dies im Vakuum erfolgen. Durch diesen Vorgang erreicht man im Allgemeinen einen quantitativen Umsatz.

Falls die Umsetzung in einem Lösungsmittel durchgeführt wird, kann das Lösungsmittel nach dem Reaktionsende durch Abdestillieren entfernt werden. Dieses kann unter Normaldruck oder erniedrigtem Druck bei Raumtemperatur oder erhöhten Temperaturen erfolgen.

Die Isolierung der gewünschten Verbindungen der allgemeinen Formel (I) kann beispielsweise auch durch Kristallisation erfolgen.

Die vorliegende Erfindung wird anhand der nachfolgenden Beispiele näher erläutert, wobei die Beispiele nicht in die Erfindung einschränkende Weise zu interpretieren sind.

### Herstellungsbeispiele:

### Beispiel 1 Herstellung von 4-[[(6-Chlorpyridin-3-yl)methyl](2,2-difluorethyl)amino]furan-2(5H)-on

Zu einer Suspension von 1,7 g N-[(6-Chlorpyridin-3-yl)methyl]-N-(2,2-difluorethyl)-4-hydroxy-2-oxo-2,5-dihydrofuran-3-carboxamid in 50 ml Butyronitril werden bei Raumtemperatur 0,5 g Kaliumhydrogensulfat zugesetzt. Die Mischung wird 5 Stunden auf Rückfluß erhitzt. Anschließend wird auf Raumtemperatur abgekühlt und mit 30 ml Wasser gewaschen. Das Lösungsmittel wird im Vakuum entfernt. Man erhält 1 g 4-[[(6-Chlorpyridin-3-yl)methyl](2,2-difluorethyl)amino]furan-2(5H)-on (dies entspricht 77 % Ausbeute).

¹H-NMR (CDCl₃, 298K) δ: 3,53 (td, 2H), 4,52 (s, 2H), 4,82 (s, 2H), 4,83 (s, 1H), 5,96 (tt, 1H), 7,37 (d, 1H), 7,55 (dd, 1H), 8,27(d, 1H)

### Beispiel 2: Herstellung von N-[(6-Chlorpyridin-3-yl)methyl]-N-(2,2-difluorethyl)-4-hydroxy-2-oxo-2,5-dihydrofuran-3-carboxamid

10 g Methyl-4-hydroxy-2-oxo-2,5-dihydrofuran-3-carboxylat werden in 111 g Butyronitril vorgelegt und mit 5 g N-[(6-Chlorpyridin-3-yl)methyl]-2,2-difluorethanamin versetzt. Die Lösung wird 3 h auf 65 °C erhitzt. Anschließend wird die Lösung mit 300 ml Wasser extrahiert und anschließend mit 300 ml 5 %iger Salzsäure-Lösung gewaschen. Die organische Phase wird über Magnesiumsulfat getrocknet und das Lösungsmittel im Vakuum entfernt. Zur Reinigung wird aus Isopropanol umkristallisiert.

NMR(CD₃CN): 1H(s, 8,19 ppm); 1H (d, 7,63 ppm); 1H (d, 7,24 ppm); 1 H (t, 6,09 ppm); 2 H (s, 4,62 ppm); 2 H (s, 3,98 ppm); 2 H (m, 3,62 ppm)

## Patentansprüche

1. Verfahren zum Herstellen von Verbindungen der Formel (I) 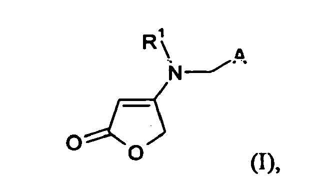 **dadurch gekennzeichnet, dass** Verbindungen der Formel (II) 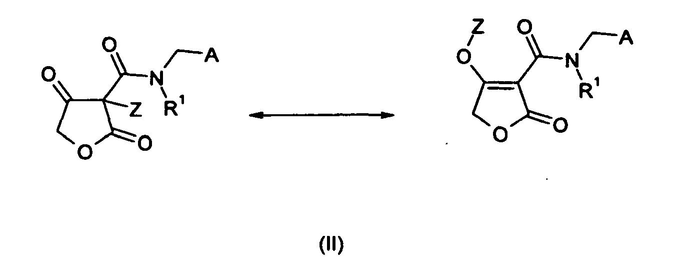 in Gegenwart einer Br⌀nstedt-Säure zu Verbindungen der Formel (I) umsetzt werden,
wobei
R¹ für Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyloxyalkyl, Halogencycloalkylalkyl oder Arylalkyl steht;
Z für Wasserstoff, Alkalimetall oder Erdalkalimetall steht; und
A für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder für
für Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder für 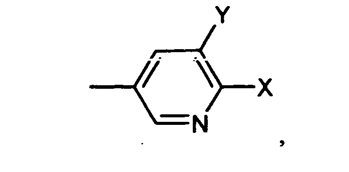 wobei
X für Halogen, Alkyl oder Halogenalkyl steht und
Y für Halogen, Alkyl, Halogenalkyl, Halogenalkoxy, Azido oder Cyan steht.

2. Verfahren nach Anspruch 1, **dadurch gekennzeichnet, dass** die Brønstedt-Säure ausgewählt ist unter H₃PO₄, H₂SO₄, HCl, HBr, HF, KHSO₄, Trifluoressigsäure, Essigsäure, Methansulfonsäure und p-Toluolsulfonsäure ist.

3. Verfahren nach Anspruch 1 oder 2, wobei
R¹ für Wasserstoff, C₁₋₁₂-Alkyl, C₁₋₂-Halogenalkyl, C₂₋₁₂-Alkenyl, C₂₋₁₂-Halogenalkenyl, C₂₋₁₂-Alkinyl, C₃₋₈-Cycloalkyl, C₃₋₈-CycloalkylC₁₋₂-alkyl, C₃₋₈-Halogencycloalkyl, C₁₋₁₂-Alkoxy, C₁₋₁₂-Alkyloxyalkyl, C₃₋₈-HalogencycloalkylC₁₋₁₂-alkyl oder C₆₋₁₄ArylC₁₋₁₂-alkyl steht;
Z für Wasserstoff, Alkalimetall oder Erdalkalimetall steht; und
A für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder für
für Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl, welcher gegebenenfalls durch Fluor, Chlor, Brom, Cyano, Nitro, C₁-C₄-Alkyl, welches gegebenenfalls durch Fluor und/oder Chlor substituiert ist, C₁-C₃-Alkylthio, welches gegebenenfalls durch Fluor und/oder Chlor substituiert ist, oder C₁-C₃-Alkylsulfonyl, welches gegebenenfalls durch Fluor und/oder Chlor substituiert ist, substituiert ist, oder für 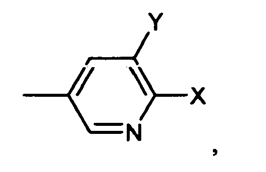 wobei
X für Chlor, Brom, Iod, Fluor, C₁₋₂-Alkyl oder C₁₋₁₂-Halogenalkyl steht und
Y für Chlor, Brom, Iod, Fluor, C₁₋₁₂-Alkyl oder C₁₋₁₂-Halogenalkyl, C₁₋₁₂-Halogenalkoxy, Azido oder Cyan steht.

4. Verfahren nach Anspruch 1 oder 2, wobei
A für 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pirid-3-yl 5-brom-6-chlor-pyrid-3-yl, 5-Fluor-6-brom 5-6-Dichlor pyrid-3-yl pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl oder 5-Difluormethyl-6-chlor-pyrid-3-yl steht;
R¹ für Methyl, Ethyl, Propyl, Vinyl, Allyl, Propargyl, Cyclopropyl, Alkoxyalkyl, 2-Fluor-ethyl, 2,2-Difluor-ethyl oder 2-Fluor-cyclopropyl steht; und
Z für Natrium, Kalium oder Wasserstoff steht.

5. Verfahren nach Anspruch 1 oder 2, wobei
A für 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl oder 5-Fluor-6-brom-pyrid-3-yl und
R¹ für Methyl, Ethyl, n-Propyl, n-Prop-2-enyl, n-Prop-2-inyl, Cyclopropyl, Methoxyethyl, 2-Fluorethyl oder 2,2-Difluor-ethyl steht; und
Z für Natrium oder Wasserstoff steht.

6. Verfahren nach einem der Ansprüche 1 bis 5, **dadurch gekennzeichnet, dass** die Umsetzung in einem Lösungsmittel durchgerührt wird, das ausgewählt ist aus der Gruppe bestehend aus Dioxan, Butyronitril, Propionitril, Acetonitril, DME, Toluol, Methyl-THF, Dichlorbenzol, Chlorbenzol, n-Heptan, iso-Butanol, n-Butanol, Ethanol, Methyl-tert.-butylether, Isopropylethylether und Mischungen davon.

7. Verfahren nach einem der Ansprüche 1 bis 6, **dadurch gekennzeichnet**, die Umsetzung in einem Temperaturbereich von 20 °C bis 150°C durchgeführt wird.

8. Verbindungen der Formel **(II)** 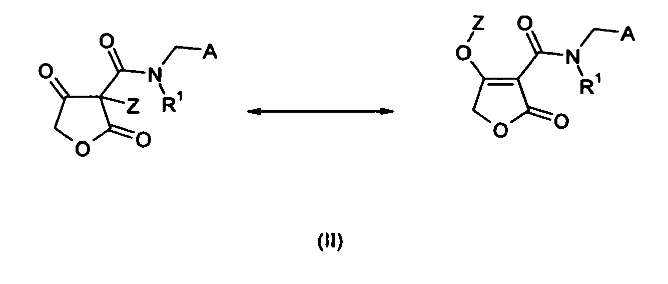 in welcher die Reste A, Z und R¹ wie in einem der Ansprüche 1 bis 5 definiert sind.

## Claims

1. Process for the preparation of compounds of the formula (I) 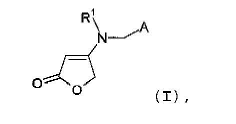 **characterized in that** compounds of the formula (II) 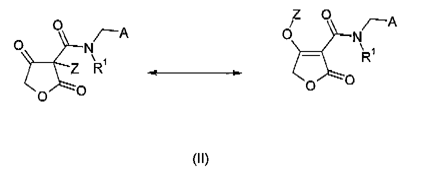 are reacted in the presence of a Brønsted acid to give compounds of the formula (I),
where
R¹ is hydrogen, alkyl, haloalkyl, alkenyl, haloalkenyl, alkynyl, cycloalkyl, cycloalkylalkyl, halocycloalkyl, alkoxy, alkyloxyalkyl, halocycloalkylalkyl or arylalkyl;
Z is hydrogen, alkali metal or alkaline earth metal; and
A is pyrid-2-yl or pyrid-4-yl or is pyrid-3-yl which is optionally substituted in position 6 by fluorine, chlorine, bromine, methyl, trifluoromethyl or trifluoromethoxy, or is pyridazin-3-yl which is optionally substituted in position 6 by chlorine or methyl, or is pyrazin-3-yl or is 2-chloropyrazin-5-yl or is 1,3-thiazol-5-yl which is optionally substituted in position 2 by chlorine or methyl, or
is pyrimidinyl, pyrazolyl, thiophenyl, oxazolyl, isoxazolyl, 1,2,4-oxadiazolyl, isothiazolyl, 1,2,4-triazolyl or 1,2,5-thiadiazolyl which is optionally substituted by fluorine, chlorine, bromine, cyano, nitro, C₁-C₄-alkyl (which is optionally substituted by fluorine and/or chlorine), C₁-C₃-alkylthio (which is optionally substituted by fluorine and/or chlorine), or C₁-C₃-alkylsulphonyl (which is optionally substituted by fluorine and/or chlorine), or is 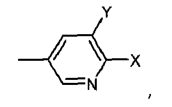 where
X is halogen, alkyl or haloalkyl and
Y is halogen, alkyl, haloalkyl, haloalkoxy, azido or cyano.

2. Process according to Claim 1, **characterized in that** the Brønsted acid is selected from H₃PO₄, H₂SO₄, HCl, HBr, HF, KHSO₄, trifluoroacetic acid, acetic acid, methanesulphonic acid and p-toluenesulphonic acid.

3. Process according to Claim 1 or 2, where
R¹ is hydrogen, C₁₋₁₂-alkyl, C₁₋₁₂-haloalkyl, C₂₋₁₂-alkenyl, C₂₋₁₂-haloalkenyl, C₂₋₁₂-alkynyl, C₃₋₈-cycloalkyl, C₃₋₈-cycloalkylC₁₋₁₂-alkyl, C₃₋₈-halocycloalkyl, C₁₋₁₂-alkoxy, C₁₋₁₂-alkyloxyalkyl, C₃₋₈-halocycloalkylC₁₋₁₂-alkyl or C₆₋₁₄-arylC₁₋₁₂-alkyl;
Z is hydrogen, alkali metal or alkaline earth metal; and
A is pyrid-2-yl or pyrid-4-yl or is pyrid-3-yl which is optionally substitution in position 6 by fluorine, chlorine, bromine, methyl, trifluoromethyl or trifluoromethoxy, or is pyridazin-3-yl which is optionally substituted in position 6 by chlorine or methyl, or is pyrazin-3-yl or is 2-chloropyrazin-5-yl or is 1,3-thiazol-5-yl which is optionally substituted in position 2 by chlorine or methyl, or
is pyrimidinyl, pyrazolyl, thiophenyl, oxazolyl, isoxazolyl, 1,2,4-oxadiazolyl, isothiazolyl, 1,2,4-triazolyl or 1,2,5-thiadiazolyl which is optionally substituted by fluorine, chlorine, bromine, cyano, nitro, C₁-C₄-alkyl which is optionally substituted by fluorine and/or chlorine, C₁-C₃-alkylthio which is optionally substituted by fluorine and/or chlorine, or C₁-C₃-alkylsulphonyl which is optionally substituted by fluorine and/or chlorine, or is 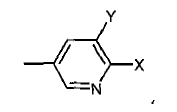 where
X is chlorine, bromine, iodine, fluorine, C₁₋₁₂-alkyl or C₁₋₁₂-haloalkyl and
Y is chlorine, bromine, iodine, fluorine, C₁₋₁₂-alkyl or c₁₋₁₂-haloalkyl, C₁₋₁₂- haloalkoxy, azido or cyano.

4. Process according to Claim 1 or 2, where
A is 6-fluoropyrid-3-yl, 6-chloropyrid-3-yl, 6-bromopyrid-3-yl, 6-chloro-1,4-pyridazin-3-yl, 2-chloro-1,3-thiazol-5-yl, 2-chloropyrimidin-5-yl, 5-fluoro-6-chloropyrid-3-yl, 5,6-dichloropyrid-3-yl, 5-bromo-6-chloropyrid-3-yl, 5-fluoro-6-bromopyrid-3-yl, 5-chloro-6-bromopyrid-3-yl, 5,6-dibromopyrid-3-yl, 5-methyl-6-chloropyrid-3-yl, 5-chloro-6-iodopyrid-3-yl or 5-difluoromethyl-6-chloropyrid-3-yl;
R¹ is methyl, ethyl, propyl, vinyl, allyl, propargyl, cyclopropyl, alkoxyalkyl, 2-fluoroethyl, 2,2-difluoroethyl or 2-fluorocyclopropyl; and
Z is sodium, potassium or hydrogen.

5. Process according to Claim 1 or 2, where
A is 6-chloropyrid-3-yl, 6-bromopyrid-3-yl, 6-chloro-1,4-pyridazin-3-yl, 2-chloro-1,3-thiazol-5-yl, 5-fluoro-6-chloropyrid-3-yl or 5-fluoro-6-bromopyrid-3-yl and
R¹ is methyl, ethyl, n-propyl, n-prop-2-enyl, n-prop-2-inyl, cyclopropyl, methoxyethyl, 2-fluoroethyl or 2,2-difluoroethyl; and
Z is sodium or hydrogen.

6. Process according to one of Claims 1 to 5, **characterized in that** the reaction is carried out in a solvent which is selected from the group consisting of dioxane, butyronitrile, propionitrile, acetonitrile, DME, toluene, methyl-THF, dichlorobenzene, chlorobenzene, n-heptane, isobutanol, n-butanol, ethanol, methyl tert-butyl ether, isopropyl ethyl ether and mixtures thereof.

7. Process according to one of Claims 1 to 6, **characterized in that** the reaction is carried out in a temperature range from 20°C to 150°C.

8. Compounds of the formula (II) 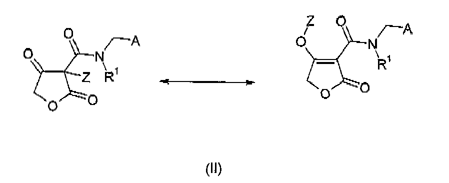 in which the radicals A, Z and R¹ are as defined in one of Claims 1 to 5.

## Revendications

1. Procédé pour la préparation de composés de formule (I) 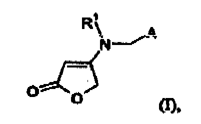 **caractérisée en ce qu'**on convertit en composés de formule (I) des composés de formule (II) 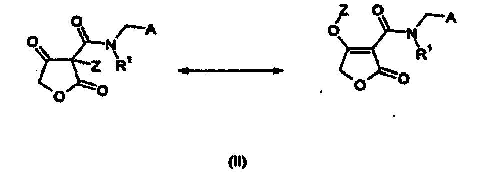 en présence d'un acide de Brønsted,
où
R¹ représente un atome d'hydrogène, un groupe alkyle, halogénoalkyle, alcényle, halogénoalcényle, alcynyle, cycloalkyle, cycloalkylalkyle, halogénocycloalkyle, alcoxy, alkyloxyalkyle, halogénocycloalkylalkyle ou arylalkyle ;
Z représente un atome d'hydrogène, un atome de métal alcalin ou alcalino-terreux ; et
A représente le groupe pyrid-2-yle ou pyrid-4-yle ou représente un groupe pyrid-3-yle qui est éventuellement substitué en position 6 par un atome de fluor, chlore, brome, par un groupe méthyle, trifluorométhyle ou trifluorométhoxy ou représente un groupe pyridazin-3-yle qui est éventuellement substitué en position 6 par un atome de chlore ou par un groupe méthyle ou représente un groupe pyrazin-3-yle ou 2-chloropyrazin-5-yle ou représente un groupe 1,3-thiazol-5-yle qui est éventuellement substitué en position 2 par un atome de chlore ou par un groupe méthyle, ou représente
un groupe pyrimidinyle, pyrazolyle, thiophényle, oxazolyle, isoxazolyle, 1,2,4-oxadiazolyle, isothiazolyle, 1,2,4-triazolyle ou 1,2,5-thiadiazolyle, qui est éventuellement substitué par le fluor, le chlore, le brome, cyano, nitro, alkyle en C₁-C₄ (qui est éventuellement substitué par le fluor et/ou le chlore), alkyl(C₁-C₃)thio (qui est éventuellement substitué par le fluor et/ou le chlore), ou alkyl(C₁-C₃)sulfonyle (qui est éventuellement substitué par le fluor et/ou le chlore), ou représente 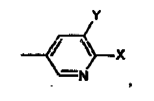 où
X représente un atome d'halogène, un groupe alkyle ou halogénoalkyle et
Y représente un atome d'halogène, un groupe alkyle, halogénoalkyle, halogénoalcoxy, azido ou cyano.

2. Procédé selon la revendication 1, **caractérisé en ce que** l'acide de Brønsted est choisi parmi H₃PO₄, H₂SO₄, HCl, HBr, HF, KHSO₄, l'acide trifluoroacétique, l'acide acétique, l'acide méthanesulfonique et l'acide p-toluènesulfonique.

3. Procédé selon la revendication 1 ou 2, dans lequel
R¹ représente un atome d'hydrogène, un groupe alkyle en C₁-C₁₂, halogénoalkyle en C₁-C₁₂, alcényle en C₂-C₁₂, halogénoalcényle en C₂-C₁₂, alcynyle en C₂-C₁₂, cycloalkyle en C₃-C₈, cycloalkyl(C₃-C₈)-alkyle(C₁-C₁₂), halogénocycloalkyle en C₃-C₈, alcoxy en C₁-C₁₂, alkyloxyalkyle(C₁-C₁₂), halogéno-cycloalkyl (C₃-C₈) alkyle (C₁-C₁₂) ou aryl (C₆-C₁₄) - alkyle(C₁-C₁₂) ;
Z représente un atome d'hydrogène, un atome de métal alcalin ou alcalino-terreux ; et
A représente le groupe pyrid-2-yle ou pyrid-4-yle ou représente un groupe pyrid-3-yle qui est éventuellement substitué en position 6 par un atome de fluor, chlore, brome, par un groupe méthyle, trifluorométhyle ou trifluorométhoxy ou représente un groupe pyridazin-3-yle qui est éventuellement substitué en position 6 par un atome de chlore ou par un groupe méthyle ou représente un groupe pyrazin-3-yle ou 2-chloropyrazin-5-yle ou représente un groupe 1,3-thiazol-5-yle qui est éventuellement substitué en position 2 par un atome de chlore ou par un groupe méthyle, ou représente un groupe pyrimidinyle, pyrazolyle, thiophényle, oxazolyle, isoxazolyle, 1,2,4-oxadiazolyle, isothiazolyle, 1,2,4-triazolyle ou 1,2,5-thiadiazolyle, qui est éventuellement substitué par le fluor, le chlore, le brome, cyano, nitre, alkyle en C₁-C₄, qui est éventuellement substitué par le fluor et/ou le chlore, alkyl(C₁-C₃)thio, qui est éventuellement substitué par le fluor et/ou le chlore, ou alkyl(C₁-C₃)sulfonyle qui est éventuellement substitué par le fluor et/ou le chlore, ou représente 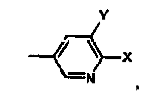
où
X représente un atome de chlore, brome, iode, fluor, un groupe alkyle en C₁-C₁₂ ou halogénoalkyle en C₁-C₁₂ et
Y représente un atome de chlore, brome, iode, fluor, un groupe alkyle en C₁-C₁₂ ou halogénoalkyle en C₁-C₁₂, halogénoalcoxy en C₁-C₁₂, azido ou cyano.

4. Procédé selon la revendication 1 ou 2, dans lequel
A représente le groupe 6-fluoro-pyrid-3-yle, 6-chloro-pyrid-3-yle, 6-bromo-pyrid-3-yle, 6-chloro-1,4-pyridazin-3-yle, 2-chloro-1,3-thiazol-5-yle, 2-chloro-pyrimidin-5-yle, 5-fluoro-6-chloro-pyrid-3-yle, 5,6-dichloro-pyrid-3-yle, 5-bromo-6-chloro-pyrid-3-yle, 5-fluoro-6-bromo-pyrid-3-yle, 5-chloro-6-bromo-pyrid-3-yle, 5,6-dibromo-pyrid-3-yle, 5-méthyl-6-chloro-pyrid-3-yle, 5-chloro-6-iodo-pyrid-3-yle ou 5-difluoro-méthyl-6-chloro-pyrid-3-yle ;
R¹ représente un groupe méthyle, éthyle, propyle, vinyle, allyle, propargyle, cyclopropyle, alcoxyalkyle, 2-fluoro-éthyle, 2,2-difluoro-éthyle ou 2-fluoro-cyclopropyle ; et
Z représente un atome de sodium, potassium ou d'hydrogène.

5. Procédé selon la revendication 1 ou 2, dans lequel
A représente le groupe 6-chloro-pyrid-3-yle, 6-bromo-pyrid-3-yle, 6-chloro-1,4-pyridazin-3-yle, 2-chloro-1,3-thiazol-5-yle, 5-fluoro-6-chloro-pyrid-3-yle ou 5-fluoro-6-bromo-pyrid-3-yle et
R¹ représente le groupe méthyle, éthyle, n-propyle, n-prop-2-ényle, n-prop-2-ynyle, cyclopropyle, méthoxyéthyle, 2-fluoroéthyle ou 2,2-difluoro-éthyle ; et
Z représente un atome de sodium ou d'hydrogène.

6. Procédé selon l'une quelconque des revendications 1 à 5, **caractérisé en ce qu'**on effectue la réaction dans un solvant qui est choisi dans le groupe constitué par le dioxane, le butyronitrile, le propionitrile, l'acétonitrile, le DME, le toluène, le méthyl-THF, le dichlorobenzène, le chlorobenzène, le n-heptane, l'isobutanol, le n-butanol, l'éthanol, l'oxyde de méthyle et de tert-butyle, l'oxyde d'éthyle et d'isopropyle et des mélanges de ceux-ci.

7. Procédé selon l'une quelconque des revendications 1 à 6, **caractérisé en ce qu'**on effectue la réaction dans une plage de température de 20 °C à 150 °C.

8. Composés de formule (II) 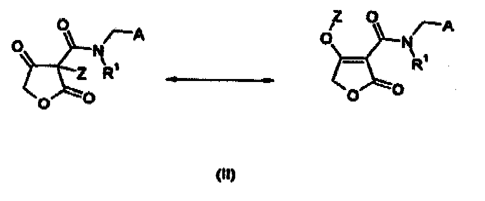 dans laquelle les radicaux A, Z et R¹ sont tels que définis dans l'une quelconque des revendications 1 à 5.
